Europäisches Patentamt

European Patent Office

Office européen des brevets

(11) Numéro de publication : **0 467 814 A1**

# (12) DEMANDE DE BREVET EUROPEEN

(21) Numéro de dépôt : **91420262.7**

(22) Date de dépôt : **16.07.91**

(51) Int. Cl.⁵ : **A61B 19/02, // A61F2/16**

(30) Priorité : **16.07.90 FR 9009709**

(43) Date de publication de la demande :
**22.01.92 Bulletin 92/04**

(84) Etats contractants désignés :
**AT BE CH DE DK ES FR GB GR IT LI LU NL SE**

(71) Demandeur : **CHRISTIAN DALLOZ S.A.**
**Lieu-dit Daillères**
**F-39200 Saint Claude (FR)**

(72) Inventeur : **Jeantot, Bernard**
**11 rue de la Mouillère**
**F-25000 Besançon (FR)**
Inventeur : **Billard, Etienne**
**44 rue Jean Wyrsch**
**F-25000 Besançon (FR)**

(74) Mandataire : **Guerre, Dominique et al**
**Cabinet Germain et Maureau 20 Boulevard**
**Eugène Deruelle BP 3011**
**F-69392 Lyon Cédex 03 (FR)**

(54) **Emballage pour un élément substantiellement plat.**

(57) Un emballage pour un élément substantiellement plat, notamment circulaire, par exemple un implant intra-oculaire (4), comprend :
un réceptacle (1) substantiellement plat constitué par un boîtier (2), et un couvercle (3), déplaçables longitudinalement l'un rapport à l'autre ;
un organe de maintien (5) de l'élément plat, présentant deux mors (6,7) en vis-à-vis, comprenant chacun au moins une griffe (11) comportant un crochet (11a) et un biais (11b) vers le bas, ledit organe de maintien (5) comprenant une charnière (8) entre les deux mors (6,7), un moyen d'actionnement en rotation, des deux mors (6,7) l'un par rapport à l'autre, autour de la charnière (8), entre deux positions, l'une de serrage dans laquelle l'élément plat (4) est bridé par les griffes (11) maintenues rapprochées, et l'autre de libération dans laquelle l'élément plat (4) est dégagé des griffes (11) et repose sur le biais (11b).
Le moyen d'actionnement en rotation comprend un moyen de serrage constitué par deux tenons (12) disposés sur le couvercle (3) ou les deux mors (6,7) respectivement, s'engageant avec les deux mors (6,7) ou ledit couvercle (3)respectivement, pour faire pivoter les deux mors (6,7) vers le bas dans la position de serrage, lors du mouvement de fermeture du réceptacle (1).

FIG_1

EP 0 467 814 A1

Jouve, 18, rue Saint-Denis, 75001 PARIS

L'invention concerne le domaine des emballages, tels qu'obtenus par moulage d'une matière plastique.

L'invention sera plus particulièrement décrite par référence à l'emballage des implants intra-oculaires, constituant des éléments plats et circulaires, de petite taille, et relativement fragiles, en tout cas en ce qui concerne la liaison de la partie haptique avec la partie optique. Mais bien entendu la présente invention ne saurait être limitée à ce secteur technique, et peut être généralisée à des éléments à conditionner de toute nature ou de toute taille.

Après son obtention, l'implant oculaire est soumis à une étape de stérilisation, puis mis à disposition du praticien pour opérer l'implantation. La grande fragilité de l'élément sera sollicitée, avant son implantation, en particulier au cours de son transport de son lieu de production ou stockage à son lieu d'utilisation. Il est donc indispensable que l'élément soit maintenu dans un réceptacle ne lui autorisant aucun mouvement. En plus, l'élément doit être très aisément accessible au moment de son utilisation.

Pour limiter les degrés de liberté d'un implant intra-oculaire dans son conditionnement, selon US-A-4 402 396, on a déjà proposé un emballage comprenant :

– un réceptacle substantiellement plat et parallélépipédique, constitué par un boîtier, et un couvercle fixé sur un plateau, le boîtier et le couvercle étant déplaçables longitudinalement l'un par rapport à l'autre par l'intermédiaire de glissières, entre deux positions, l'une d'ouverture dans laquelle l'utilisateur peut accéder à l'intérieur du réceptacle, et l'autre de fermeture dans laquelle le couvercle obture complètement le boîtier ;

– un organe de maintien de l'élément plat, intégré au boîtier, comprenant deux mors en vis-à-vis, comportant chacun une griffe comportant un crochet et un biais ; cet organe de maintien comprenant une charnière entre les deux mors, orientée selon la direction longitudinale de déplacement du couvercle par rapport au boîtier ;

– un élément strié fixé à une extrémité du boîtier pour permettre de déplacer longitudinalement le boîtier à l'intérieur du couvercle ;

– un moyen d'actionnement en rotation des deux mors l'un par rapport à l'autre, autour de la charnière, entre deux positions, l'une de serrage dans laquelle l'élément plat est bridé par les griffes maintenues rapprochées, et l'autre de libération dans laquelle l'élément plat est dégagé des griffes et repose sur le biais, ce moyen d'actionnement en rotation comprenant une rampe médiane longitudinale prévue sur le plateau, s'engageant sous la charnière entre les deux mors pour faire pivoter les deux mors vers le haut, autour de ladite charnière, au cours du mouvement d'ouverture du réceptacle et libérer l'implant.

Un emballage tel que précédemment défini est complexe quant à sa structure et son agencement, et sa réalisation, notamment en matière plastique moulée ou injectée, s'avère difficile ou délicate.

La présente invention a pour objet un emballage du type précédemment défini, aisément réalisable, se présentant simplement sous la forme d'un réceptacle traditionnel, notamment plat et rectangulaire, et obtenu simplement par la coopération avec glissières d'un couvercle sur un boîtier.

Selon la présente invention, l'organe de maintien de l'élément plat est distinct du boîtier et contenu dans ce dernier. Et de manière complémentaire, le moyen d'actionnement en rotation comprend :

– un moyen de serrage constitué par deux tenons, disposés au choix sur le couvercle ou les deux mors respectivement, s'engageant avec les deux mors ou avec ledit couvercle respectivement, pour faire pivoter les deux mors vers le bas, autour de la charnière, dans la position de serrage, lors du mouvement de fermeture du réceptacle, par déplacement du boîtier par rapport au couvercle ;

– et/ou un moyen de libération constitué par deux doigts, disposés au choix sur le couvercle ou les deux mors respectivement, s'engageant avec les deux mors ou avec ledit couvercle respectivement, pour faire pivoter les deux mors vers le haut, autour de la charnière, dans la position de libération, lors du mouvement d'ouverture du réceptacle, toujours par déplacement du boîtier par rapport au couvercle.

La présente invention est maintenant décrite par référence aux dessins annexés, dans lesquels :

– la figure 1 représente en perspective un emballage conforme à la présente invention, en cours d'ouverture ;

– la figure 2 représente le même emballage, toujours en perspective, complètement ouvert ;

– la figure 3 représente une vue en coupe, selon la ligne de coupe IV-IV de l'emballage selon figures 1 et 2, en position fermée ;

– la figure 4 représente une vue en coupe, toujours selon la ligne IV-IV, de l'emballage selon figures 1 et 2, en positon ouverte.

Un emballage conforme à l'invention comprend principalement :

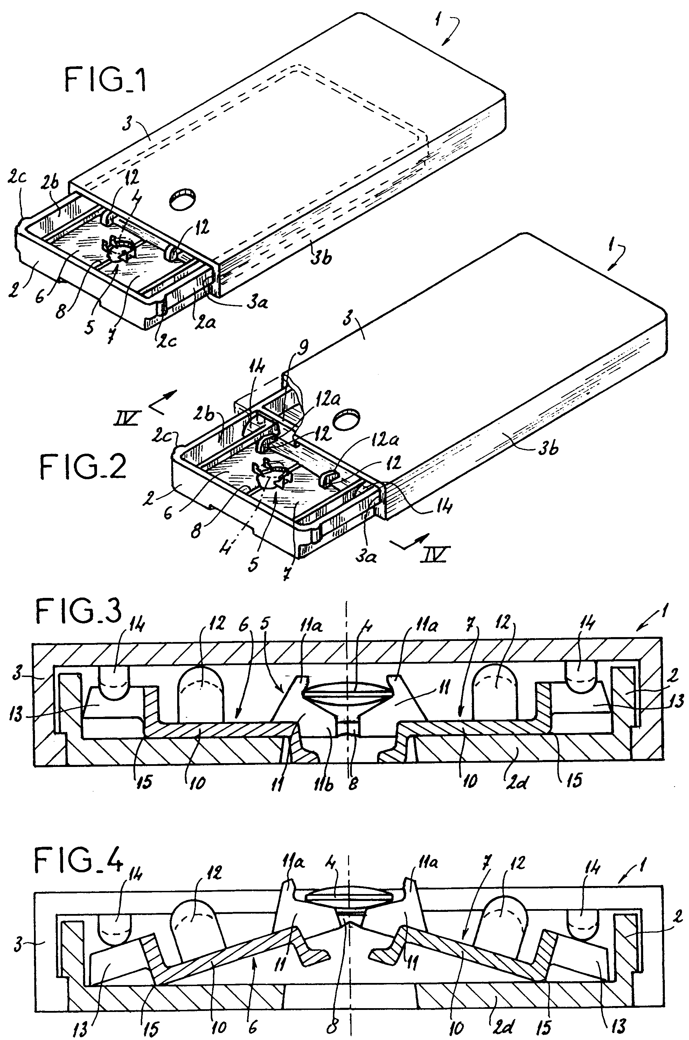

– un réceptacle 1, de forme aplatie et parallélépipédique, constitué par un boîtier 2, et un couvercle 3, monté de manière coulissante sur le boîtier 2, ces deux éléments 2 et 3 étant déplaçables l'un par rapport à l'autre, entre deux positions, j'une d'ouverture (figure 2) dans laquelle l'utilisateur peut accéder à l'intérieur du réceptacle 1 et saisir dans le boîtier 2 l'implant stocké 4, et l'autre de fermeture (vue en coupe selon figure 3) dans laquelle le couvercle 3 obture complètement le boîtier 2 ;

– un organe de maintien 5 de l'implant 4, disposé au sein du réceptacle 1, distinct du boîtier 2 ou du couvercle 3 ; cet organe 5 disposé dans le boîtier 2 comprend deux mors 6 et 7 en vis-à-vis, pouvant pivoter l'un par rapport à l'autre autour d'une charnière 8, entre deux positions, l'une (cf figure 3) de serrage dans laquelle les deux mors 6 et 7 sont engagés avec l'implant 4, et l'autre de libération (cf figure 4) dans laquelle les deux mors 6 et 7 sont dégagés de l'élément plat 4 ; la charnière 8 est orientée selon la direction longitudinale de déplacement du couvercle 3 par rapport au boîtier 2.

S'agissant du réceptacle 1, celui-ci est construit à la manière d'un étui. A cette fin, le boîtier 2 comporte deux bordures 2a supérieures, selon sa longueur, et à l'aplomb de ses deux parois latérales 2b. De manière correspondante et pour coopérer avec ces deux bordures 2a, le couvercle 3 comporte deux bordures latérales 3b, toujours selon la longueur du réceptacle 1, ayant en section transversale la forme d'un crochet, de manière à ménager deux rainures 3a coopérant chacune avec une bordure 2a. Deux pions 2c sont disposés aux deux extrémités des deux bordures 2a, pour s'accrocher de manière élastique dans deux dépressions, non représentées, prévues de manière correspondante à l'extrémité des deux rainures 3a, ceci pour bloquer le couvercle 3 sur le boîtier 2 dans sa position de fermeture. L'organe de maintien 5 présente également un encombrement relativement plat, de manière à se loger dans le volume du boîtier 2, dans un compartiment prévu à cet effet, et limité par une cloison transversale 9. La charnière 8 est disposée selon l'axe de symétrie longitudinale du réceptacle 1. Cet organe de maintien 5 est obtenu de manière monobloc en une matière plastique moulée, avec la charnière 8 constituée par un voile aminci de plastique.

Les deux mors 6 et 7 ont une constitution identique, et sont symétriques par rapport à un plan vertical passant par l'axe de la charnière 8.

Chaque mors 6 ou 7 comprend :

– un socle plat 10, pouvant reposer sur la paroi inférieure 2d du boîtier 2 (cf figure 3) ;

– deux griffes 11, saillant du socle 10 vers le haut, espacées l'une de l'autre et disposées du côté intérieur du socle 10 ; chaque griffe 11 comporte, du haut vers le bas, un crochet 11a et un biais 11b dirigés vers l'intérieur et vers le bas du boîtier ;

– un tenon 12 saillant à partir du socle 10, dirigé vers le couvercle 3, comportant à son extrémité libre une rampe oblique 12a dirigée vers le bas, et faisant un angle aigu avec la partie verticale du même tenon ; comme décrit ci-après chaque tenon coopère avec le couvercle 3 ;

– une rampe 13 dirigée vers le haut, située du côté extérieur et postérieur du mors 6 ou 7 ; comme décrit ci-après, chaque rampe 13 coopère avec un doigt 14 prévu en correspondance sur le couvercle 3, les deux doigts étant situés sur les deux côtés extérieurs respectivement, et à l'intérieur du couvercle 3 ;

– chaque mors 6 ou 7 comportant par ailleurs une zone d'appui ou décrochement 15, permettant un basculement du mors 6 ou 7, comme décrit ci-après.

En liaison avec l'ouverture-fermeture du réceptacle 1, l'organe de maintien 5 est susceptible de prendre deux positions, par pivotement des deux mors 6 ou 7 autour de la charnière 8, à savoir :

– une position de serrage (cf figure 3), dans laquelle les deux mors 6 et 7 sont engagés avec l'implant 4, plus précisément dans laquelle ce dernier est bridé uniquement par les crochets 11a des quatres griffes 11 ; dans cette position, les socles plats 10 des deux mors 6 et 7 sont situés dans un même plan ;

– et une position de libération, dans laquelle les deux mors 6 et 7 sont dégagés de l'implant 4, et plus précisément dans laquelle l'implant est dégagé des crochets 11a et repose sur les biais 11b des quatre griffes 11.

A partir de la position fermée du réceptacle, représentée à la figure 3, et dans le sens de l'ouverture du boîtier 2 par rapport au couvercle 3, les doigts 14 viennent en appui contre les rampes 13 respectivement, et poussent ces dernières vers le bas. Grâce à leur zone d'appui ou décrochement 15, les deux mors 6 et 7 sont alors basculés vers le haut, avec rotation autour de la charnière 8, pour aboutir à la position représentée à la figure 4. De cette manière, l'implant 4 est présenté vers le haut du boîtier, en étant libéré des crochets 11a, sans possibilité d'échappement, compte tenu de la disposition des griffes 11, décrites précédemment.

Dans le sens de la fermeture du couvercle 3, par rapport au boîtier 2, les doigts 14 se dégagent des rampes 13, dans un premier temps. Puis, dans un deuxième temps, le couvercle 3 s'engage avec les rampes 12a des tenons 12, en repoussant vers le bas les mors 6 et 7, toujours avec rotation autour de la charnière 8, pour aboutir à la position à plat représentée à la figure 3. Dans cette position, l'implant 4 se trouver bridé uniquement par les crochets 11a des griffes 11, de manière positive, sans contact avec les autres parties de l'emballage.

**Revendications**

**1 -** Emballage pour un élément substantiellement plat, notamment circulaire, par exemple un implant intra-oculaire, comprenant :

– un réceptacle (1) substantiellement plat constitué par un boîtier (2), et un couvercle (3), déplaçables longitudinalement l'un rapport à l'autre par l'intermédiaire de glissières, entre deux positions, l'une d'ouverture dans laquelle l'utilisateur peut accéder à l'intérieur du réceptacle, et l'autre de fermeture dans laquelle le couvercle obture complètement le boîtier ;

– un organe de maintien (5) de l'élément plat, présentant deux mors (6,7) en vis-à-vis, comprenant chacun au moins une griffe (11) comportant un crochet (11a) et un biais (11b) vers le bas, ledit organe de maintien (5) comprenant une charnière (8) entre les deux mors, orientée selon la direction longitudinale de déplacement du couvercle par rapport au boîtier ;

– un moyen d'actionnement en rotation, des deux mors l'un par rapport à l'autre, autour de la charnière et sous l'effet de déplacement longitudinal du couvercle par rapport au boîtier, entre deux positions, l'une de serrage dans laquelle l'élément plat est bridé par les griffes maintenues rapprochées, et l'autre de libération dans laquelle l'élément plat est dégagé des griffes et repose sur le biais ;

caractérisé en ce que, l'organe de maintien (5) de l'élément plat étant distinct du boîtier (2) et contenu dans ce dernier, le moyen d'actionnement en rotation comprend un moyen de serrage constitué par deux tenons (12) disposés sur le couvercle ou les deux mors (6,7) respectivement, s'engageant avec les deux mors ou ledit couvercle respectivement, pour faire pivoter les deux mors vers le bas dans la position de serrage, lors du mouvement de fermeture du réceptacle.

**2 -** Emballage selon la revendication 1, caractérisé en ce que chaque tenon (12) est solidaire d'un mors et comporte une rampe (12a) dirigée vers le bas.

**3 -** Emballage selon la revendication 1, caractérisé en ce que le moyen d'actionnement en rotation comprend aussi un moyen de libération constitué par deux doigts (14) disposés sur le couvercle (3) ou les deux mors (6,7) respectivement, s'engageant avec les deux mors ou ledit couvercle respectivement, pour faire pivoter ces derniers vers le haut dans la position de libération, lors du mouvement d'ouverture du réceptacle (1).

**4 -** Emballage selon la revendication 3, caractérisé en ce que les deux doigts (14) sont disposés sur le couvercle et les deux mors (6,7) comprennent deux rampes (13) respectivement, dirigées vers le haut, s'engageant avec les deux doigts (14) respectivement, chaque mors comportant une zone d'appui (15) par basculement contre la paroi du boîtier (2d).

FIG_1

FIG_2

FIG.3

FIG.4

Office européen
des brevets

**RAPPORT DE RECHERCHE EUROPEENNE**

Numero de la demande

EP  91 42 0262

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | US-A-4 402 396  (GRAHAM)<br>* En entier *<br>--- | 1,2,4 | A 61 B  19/02  //<br>A 61 F   2/16 |
| A | US-A-4 269 307  (LAHAYE)<br>* Colonne 4, lignes 23-61; figures 4-6 *<br>--- | 1 | |
| A | FR-A-2 620 687  (ALLERGAN)<br>* Page 11, lignes 26-31; page 12, lignes 13-28; page 13, ligne 10 - page 14, ligne 7; page 15, ligne 31 - page 16, ligne 10; figures *<br>--- | 1-4 | |
| A | US-A-4 736 836  (ALONGI)<br>----- | | |
| | | | **DOMAINES TECHNIQUES RECHERCHES (Int. Cl.5)**<br><br>A 61 F |

Le présent rapport a été établi pour toutes les revendications

| Lieu de la recherche | Date d'achèvement de la recherche | Examinateur |
|---|---|---|
| LA HAYE | 11-10-1991 | KLEIN C. |

EPO FORM 1503 03.82 (P0402)